# FASCICULE DE BREVET EUROPEEN

(11) **EP 3 707 583 B1**
(45) Date de publication et mention de la délivrance du brevet: **08.12.2021**
(21) Numéro de dépôt: 18811151.2
(22) Date de dépôt: 08.11.2018
(51) Int. Cl.: G06F 3/01, G06F 3/0354, A61B 34/00, A61B 34/35

(54) **DISPOSITIF DE RETOUR HAPTIQUE**
VORRICHTUNG MIT HAPTISCHER RÜCKMELDUNG
HAPTIC FEEDBACK DEVICE

(30) Priorité: 08.11.2017 FR 1760477
(43) Date de publication de la demande: 16.09.2020
(73) Titulaire: Centre National de la Recherche Scientifique, 75016 Paris (FR); Université de Franche-Comté, 25030 Besançon Cedex (FR)
(72) Inventeur: ANDREFF, Nicolas, 25480 Ecole-Valentin (FR); VIALLON, Marc, 25500 Morteau (FR); LESCANO, Sergio, 25000 Besancon (FR)
(74) Mandataire: Osha Liang
(86) Numéro de dépôt international: PCT/EP2018/080541
(87) Numéro de publication internationale: WO 2019/092063

(56) Documents cités:
- US-A1- 2016 188 015
- US-A1- 2017 269 691

## Description

### DOMAINE TECHNIQUE

L'invention concerne un dispositif de retour haptique, c'est-à-dire un dispositif apte à transmettre à un utilisateur un retour (à distance) en termes de vibrations, de mouvements, de changements de température, etc. lui permettant de ressentir une texture, une forme, une force, de la chaleur, etc.. L'invention concerne particulièrement un dispositif de retour haptique permettant de transmettre un retour quant au relief et à la raideur d'un environnement ou d'un objet.

L'invention peut trouver des applications dans les domaines de :
- la chirurgie robotisée, domaine dans lequel il est nécessaire d'avoir la précision la plus élevée possible, particulièrement dans le domaine de la microchirurgie assistée ;
- l'imagerie médicale, qui permet notamment de collecter, mesurer et enregistrer des informations dans un système informatique, lesquelles informations peuvent être récupérées à distance par un utilisateur ;
- la conception assistée par ordinateur, notamment pour le design, la fabrication de mobiliers, pour ressentir les formes, le confort...
- la téléopération, notamment dans les domaines du nucléaire pour lesquelles les opérations se font à distance ;
- l'activité de soutien à la rééducation ;
- l'aide aux malvoyants ;
- les jeux, notamment les jeux vidéo...,
et plus généralement dans tous les cas où l'on souhaite créer une interface matérielle active indirecte entre la main d'un utilisateur et un environnement réel, de réalité augmentée ou virtuelle ou un objet.

### ARRIERE PLAN

L'invention concerne plus particulièrement les cas où l'on souhaite créer une interface matérielle (ou physique) entre la main d'un utilisateur et une surface connectée avec un système apte à contenir des informations, par exemple des informations sur un environnement, sur un objet inanimé ou animé (par exemple, des plantes, des animaux, des champs (magnétiques, électriques ...). La surface peut être un écran et le système une tablette, un smartphone, un ordinateur ou leurs équivalents. Les informations peuvent être transformées en données numériques.

L'écran est préférentiellement tactile. On pourra parler par la suite de surface activable. Dans ce cas, une couche active est disposée sur l'écran. Cette couche active comporte une surface sensible qui peut être activée par le doigt ou la main d'un utilisateur, ou tout autre moyen d'actionnement qui puisse exciter cette couche active (des moyens électriquement conducteurs, des moyens mécaniques par triangulation ...). Une telle interface peut également présenter à l'utilisateur un retour d'information, par exemple un retour d'information sur l'environnement ou l'objet ...

La sensibilité du toucher chez l'être humain est le résultat d'un mécanisme complexe dans lequel plusieurs modalités sont impliquées. Dans le cadre de la présente invention, on s'intéresse aux systèmes haptiques, qui permettent de délivrer à l'utilisateur des informations sur un environnement ou un objet en donnant un retour physiologique sur cet environnement ou cet objet. Lorsqu'il s'agit d'un retour de force, les systèmes haptiques font intervenir le toucher physique, encore appelé kinesthésique, c'est à dire en relation avec le système nerveux et musculaire de l'opérateur. Lorsqu'il s'agit d'un état de surface, d'une texture, d'un relief, les systèmes haptiques font intervenir les récepteurs sensoriels situés à l'extrémité des terminaisons nerveuses de la peau, appelés récepteurs paciniens.

Comme indiqué par exemple dans les publications:
- «Telerobotics response requirements (IEEE international conférence on systems Man and Cybernetics Brooks 1990) » ;
- « Psychology of Touch and Blindness (Morton A. Heller, Edouard Gentaz 2013) » ;
- « Dictionnaire sensagent (http://dictionnaire.sensagent.leparisien.fr/haptique/fr-fr/) »,
l'être humain est capable de ressentir des vibrations en dessous d'une fréquence de rafraîchissement de l'ordre de 10 kHz, et une variation de force (par exemple de raideur d'un matériau touché) entre environ 30 et 300 Hz. Et au contraire, la perception du mouvement (par exemple sentir le relief, la texture, plus généralement un état de surface) est plus lente : 1 Hz si le mouvement est inattendu, 10 Hz pour les réflexes.

En d'autres termes, la sensibilité de l'être humain au retour de force est rapide, c'est-à-dire de quelques millisecondes, et il n'est pas nécessaire de transmettre une information de grande amplitude à l'utilisateur. Pour s'accorder avec cette contrainte physique, il faut donc disposer d'un système qui réagisse très rapidement (quelques millisecondes), mais qui ne délivre pas nécessairement une grande course, voire qui délivre une information autre qu'un déplacement. Et en revanche, la sensibilité de l'être humain aux changements de forme (et donc de relief) est relativement lente (quelques dixièmes de secondes), et ce, d'autant plus si les variations sont importantes, or il est nécessaire de transmettre une information de « grande » amplitude à l'utilisateur. Pour s'accorder avec cette contrainte physique, il faut donc disposer d'un système qui puisse avoir une « grande » course relativement à la main d'un utilisateur (c'est-à-dire de quelques millimètres), mais qui ne réagisse pas nécessairement très rapidement (quelques dixièmes de secondes).

Il n'est donc pas évident de répondre à la double contrainte (qui plus est contradictoire) à laquelle on est confronté lorsqu'on souhaite un retour en terme de relief (ou de forme) et de force (ou de raideur), lorsqu'on souhaite donc avoir dans un même dispositif un retour sur des informations très différentes voire contradictoires, surtout avec l'objectif de disposer d'un dispositif peu encombrant.

Par exemple, dans le domaine de la microchirurgie assistée, et plus particulièrement la microchirurgie par laser des cordes vocales (ou phono-chirurgie), l'objectif est de guider un faisceau laser de manière précise vers les cordes vocales. La phono-chirurgie ainsi appelée est définie comme suit : « toutes les procédures chirurgicales qui maintiennent, rétablissent ou améliorent la voix humaine », ou encore « la science de manipuler les éléments vibratoires du larynx afin de restaurer la fonction vocale ». Cela implique l'excision de la masse tissulaire qui pourrait être une masse bénigne ou une lésion cancéreuse. Dans la chirurgie au laser, l'utilisation du scalpel est remplacée par l'utilisation de technologies laser. Il existe une variété de technologies laser.

La méthodologie traditionnelle répandue pour le contrôle à distance du laser chirurgical est un manipulateur mécanique, avec un microscope chirurgical positionné de manière à visualiser les cordes vocales à traiter. Ce manipulateur mécanique ainsi que la source laser peuvent être situés à environ 400 mm des cordes vocales.

Plus récemment, ont été développés des systèmes chirurgicaux téléopérés basé sur un microrobot en tant que système terminal d'un endoscope souple réglable (ou laryngoscope souple dans le cas des cordes vocales) pour insérer le microrobot et des caméras à l'intérieur du corps du patient, éliminant le besoin du microscope chirurgical, et permettant d'accéder aux zones non accessibles par la technique précédente. Ces nouveaux systèmes de microchirurgies laser offrent une meilleure accessibilité et une meilleure précision que les systèmes précédents.

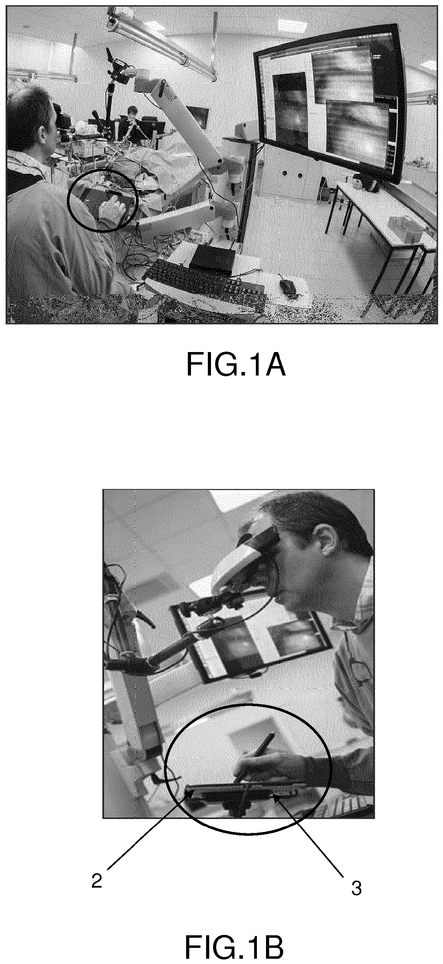

Une interface « chirurgien - microrobot » fournit en général au chirurgien un retour visuel sur une tablette, sous la forme d'images et/ou de photographies. Celui-ci peut également interagir au moyen de la tablette et d'un stylet pour tracer les trajectoires du laser sur les cordes vocales, comme on peut le voir sur les figures 1A et 1B.

Le problème de cette interface est que le chirurgien ne dispose pas d'une perception de la texture du tissu humain, comme il en dispose lorsqu'il intervient directement sur ledit tissu. Ce manque de perception concerne à la fois le relief qui peut par exemple donner une information sur la forme en 3D du tissu (remplaçant la vue) et la raideur qui peut donner par exemple une information sur le type du tissu et d'organe (remplaçant le toucher, la palpation).

L'objectif de l'invention est donc non seulement de visualiser et de tracer à distance les trajectoires que le système terminal de l'endoscope devra effectuer, mais aussi de ressentir la texture des images à distance. Par les termes « ressentir la texture des images », il faut comprendre que l'utilisateur doit pouvoir ressentir la raideur des tissus, mais aussi leurs reliefs par exemple les bosses et les cavités des tissus, et donc aussi la raideur de ces reliefs tout ceci à travers un unique dispositif tel un stylet.

Ce problème concerne tout utilisateur souhaitant ressentir à distance un environnement ou un objet dans lequel ou sur lequel il intervient.

Pour répondre à ce besoin, on connaît déjà des dispositifs qui permettent à un utilisateur de recevoir un retour sur une texture (ou un état de surface), par l'utilisation d'un dispositif haptique comportant un dispositif de vibration pour transmettre une information à un utilisateur, comme par exemple dans la demande de brevet US2015212578 qui divulgue un outil tactile, par exemple un stylet, comprenant un dispositif haptique couplé à au moins un contrôleur et un capteur, couplé au contrôleur, qui détecte lorsque l'outil tactile entre en contact avec une surface. Le contrôleur fournit une rétroaction haptique via le dispositif haptique pour simuler une texture de la surface lorsque l'outil tactile entre en contact avec la surface. En outre, l'outil comprend un dispositif de vibration et le contrôleur fait que le dispositif de vibration vibre plus fortement pour simuler une texture plus rugueuse et plus faiblement pour simuler une texture plus lisse. D'autres exemples de dispositifs haptiques sont décrits dans les documents de brevet US2017269691 et US20160188015.

Les vibreurs peuvent comprendre des actionneurs de type « linear voice coil » (en anglais) ou « actionneur à bobine linéaire mobile » (en français) dans lesquels une bobine coulisse autour d'un aimant pour transmettre une vibration à une surface tactile du dispositif, comme par exemple dans le brevet US7265750.

Alternativement, les actionneurs peuvent comprendre un induit coulissant entre deux bobines, l'alimentation alternée des bobines permettant d'attirer l'induit vers l'une ou l'autre des bobines, créant ainsi un effet oscillant pouvant générer une vibration. L'actionneur est relié à une pièce pour générer un retour haptique dans une zone de déplacement du doigt d'un utilisateur comme dans le brevet FR2927709.

Aucun de ces dispositifs ne permet de recevoir un retour à la fois sur le relief (ou la texture) et la raideur.

Il existe des dispositifs de réalité virtuelle permettant de voir en relief, mais ces dispositifs sont en général encombrants, et ils ne conviennent pas aux applications visées en particulier pour un chirurgien, d'autant qu'il faut les compléter par un dispositif haptique de retour de force. Il faudrait donc utiliser deux dispositifs différents, ce qui est encore plus gênant pour un utilisateur qui doit faire des gestes précis.

L'objectif de l'invention est de répondre aux inconvénients des dispositifs de retour haptique de l'art antérieur.

Un objectif est de proposer un dispositif de retour haptique qui permette d'avoir un retour à la fois sur relief et la raideur d'un objet ou d'un environnement réel, virtuel ou de réalité augmentée et qui soit léger, transportable, peu encombrant, et compatible avec les surfaces de visualisation tels que les écrans tactiles.

### EXPOSE DE L'INVENTION

Pour résoudre le problème précité, l'invention a pour objet un dispositif de retour haptique comprenant :
- un corps apte à former une interface avec un utilisateur ;
- des moyens de contact aptes à établir un contact avec une surface d'un système apte à contenir des informations ;
- un premier actionneur relié aux moyens de contact et apte à appliquer un mouvement de translation auxdits moyens de contact par rapport audit corps ;
- des moyens de freinage aptes à appliquer une résistance mécanique au mouvement relatif entre les moyens de contact et le corps ;
- un second actionneur apte à faire varier la résistance mécanique appliquée par les moyens de freinage.

Par direction longitudinale, il faut comprendre la direction de translation entre le corps du dispositif et les moyens de contact.

La surface du système est de manière préférentielle une surface activable.

Selon un mode de réalisation, le dispositif a sensiblement la forme d'un stylet. Cette forme a pour avantage d'être ergonomique, légère et aisée à manier.

Selon un mode de réalisation, le corps présente au moins une partie conductrice.

Selon un mode de réalisation, le corps est apte à contenir tout ou partie du premier actionneur et/ou des moyens de freinage et/ou du second actionneur.

Selon un mode de réalisation, le corps comprend une coque apte à contenir le premier actionneur, le second actionneur, et les moyens de freinage.

Selon un mode de réalisation, les moyens de contact comprennent une pointe dont une première extrémité est apte à établir un contact avec une surface. Une pointe permet en outre de rendre plus précis le toucher de la surface.

Selon un mode de réalisation, la première extrémité de la pointe est apte à exciter la surface.

Selon un mode de réalisation, le premier actionneur comprend un moteur linéaire.

Selon un mode de réalisation, le dispositif comprend des premiers moyens de liaison aptes à relier le premier actionneur aux moyens de contact.

Selon un mode de réalisation, le dispositif comprend des seconds moyens de liaison aptes à relier le second actionneur aux moyens de freinage.

Selon un mode de réalisation, les moyens de freinage comprennent un ressort. Un ressort est aisé à mettre en œuvre, aisément modulable et sa raideur peut être variée facilement en jouer sur la longueur du ressort.

Selon un mode de réalisation particulier, le second actionneur est apte à faire varier la raideur du ressort en faisant varier la longueur dudit ressort.

Selon un mode de réalisation particulier, le second actionneur comprend un moteur rotatif.

Selon un mode de réalisation particulier, les moyens de liaison comprennent une liaison de type pignon - crémaillère.

Selon un mode de réalisation, le dispositif comprend en outre des moyens d'alimentation, aptes à connecter électriquement les premier et second actionneurs dudit dispositif.

Selon un mode de réalisation, les moyens d'alimentation comprennent une batterie.

Selon un mode de réalisation, les moyens d'alimentation (60) comprennent une interface de branchement avec une alimentation extérieure.

Selon un mode de réalisation, les moyens d'alimentation sont aptes à activer électriquement les moyens de contact.

Selon un mode de réalisation, le dispositif comprend en outre des moyens de traitement aptes à activer le premier actionneur et le second actionneur en fonction de la position des moyens de contacts sur la surface.

### BREVE DESCRIPTION DES FIGURES

D'autres caractéristiques et avantages de l'invention apparaîtront à l'aide de la description qui suit donnée à titre illustratif et non limitatif, faite en regard des figures annexées parmi lesquels :
- les figures 1A et 1B illustrent une interface de travail entre un chirurgien et un microrobot comprenant une tablette et un stylet selon l'art antérieur ;
- la figure 2 donne un schéma de principe du dispositif selon un mode de réalisation ;
- les figures 3A et 3B illustrent un dispositif selon le même mode de réalisation, avec plus de détails, et selon une vue en coupe et une vue en 3D ;
- la figure 4 montre le même mode de réalisation du dispositif en vue extérieure ;
- la figure 5 illustre et précise le fonctionnement d'un dispositif selon le même mode de réalisation.

### EXPOSÉ DÉTAILLÉ DE MODES DE RÉALISATION PARTICULIERS

La figure 1 a déjà été décrite et ne sera pas reprise ici.

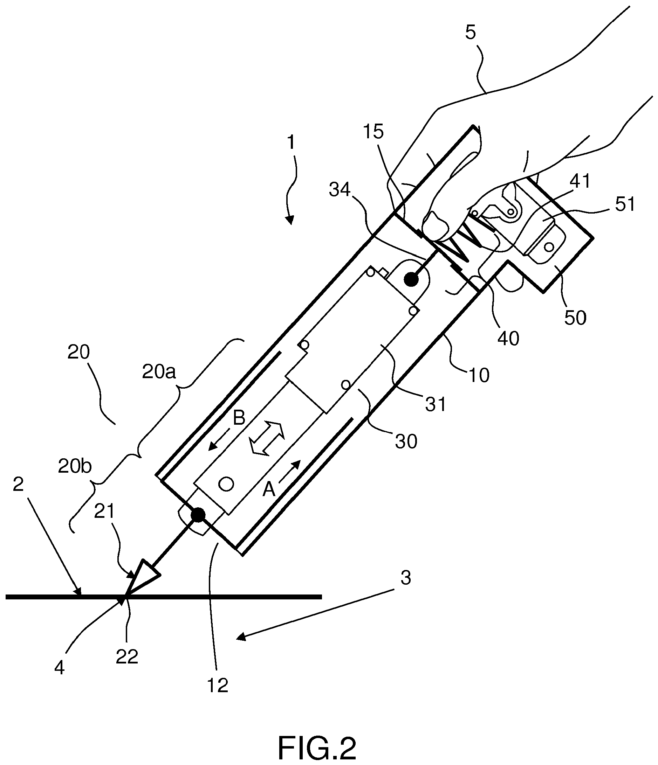

Les figures 2, 3A, 3B, 4 et 5 illustrent le même mode de réalisation, certaines figures donnant plus de détails de réalisation (détails mécaniques, ou électroniques, ou de fonctionnement).

La figure 2 montre un schéma de principe du dispositif selon l'invention. Le dispositif 1 de retour haptique comprend un corps 10. Une première extrémité 11 du corps peut être tenue par la main 5 de l'utilisateur, assurant ainsi l'interface avec ce dernier. La première extrémité 11 du corps peut présenter une forme ergonomique apte à favoriser une bonne prise et/ou une bonne transmission des mouvements à la main de l'utilisateur.

Le dispositif comprend également des moyens de contact 20 présentant une pointe 21.

Lorsqu'on pointe sur une surface activable telle une surface 2 tactile d'un système 3 apte à contenir des données (une tablette, un smartphone, un ordinateur...), il faut prévoir un mode d'excitation de ladite surface. Par exemple, l'excitation peut être capacitive, inductive, mécanique ou thermique.

La pointe 21 doit donc être apte à exciter la surface activable. Par exemple, comme illustré, elle peut comprendre une partie 22 apte à exciter ladite surface activable. Ainsi, la pointe 21, via la partie 22, permet d'établir une connexion pour exciter la surface activable.

Selon l'exemple représenté, l'excitation est électrique et la pointe 21 comprend une partie 22 électriquement conductrice. Alternativement, la pointe 21 peut être entièrement conductrice.

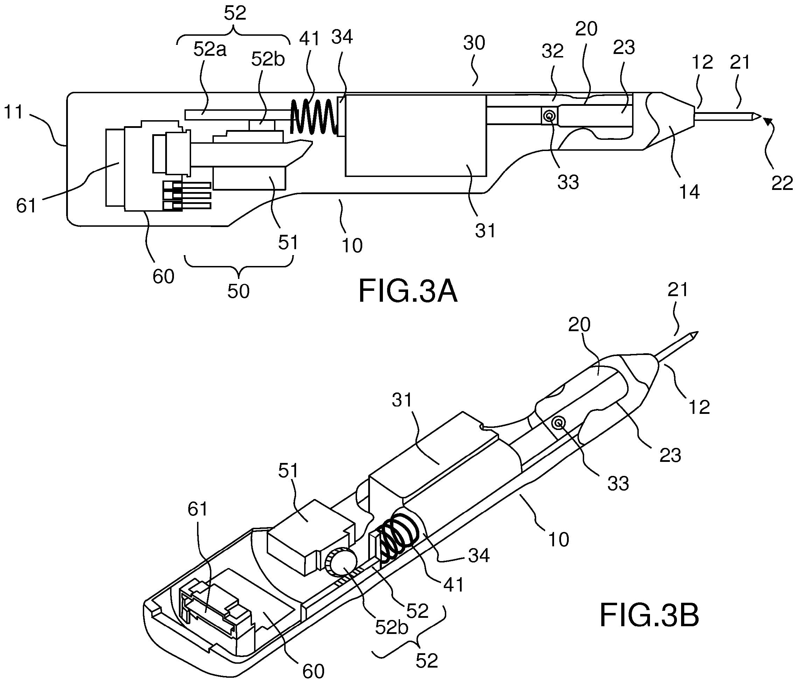

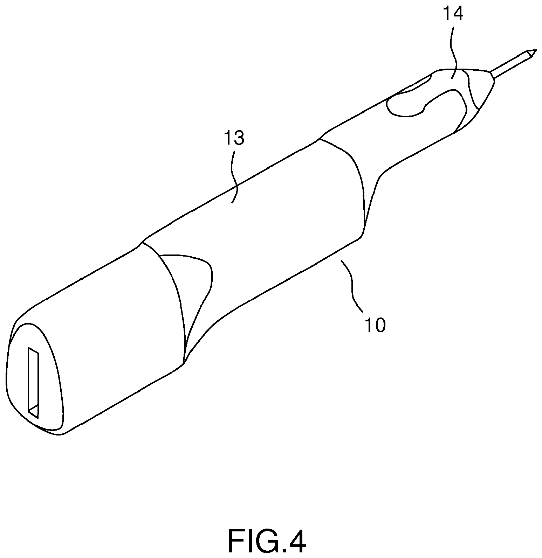

Alternativement ou en complément, le corps 10 peut comprendre une partie conductrice 14 représentée sur les figures 3A à 3B et 4. Ladite partie conductrice du corps 10 est prévue pour faciliter l'excitation de la surface activable par l'existence même d'une tension électrique, même minime, du corps humain (de l'utilisateur).

L'excitation électrique nécessite en effet une continuité électrique pour pouvoir communiquer avec le système 3. Les pointes 21 du commerce utilisent couramment la tension électrique du corps humain transmise par la main 5 et le contact électrique entre la pointe 21 et la main 5 (d'ailleurs la main suffit à établir la continuité).

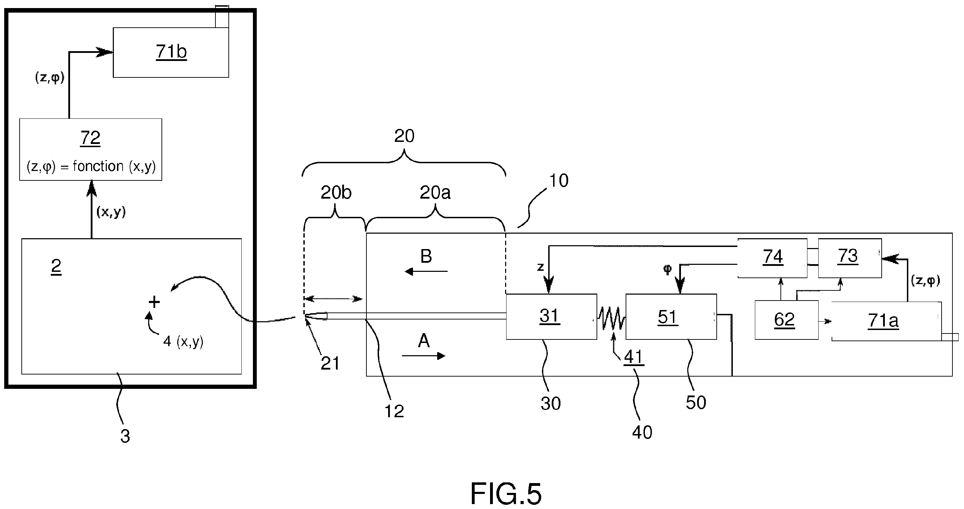

Alternativement, la pointe 21 peut être connectée à un système électrique. Cette connexion électrique peut être réalisée par l'intermédiaire des moyens d'alimentation 60 qui sont décrits en relation avec les figures 3A et 3B, par exemple une batterie 62, représentée en figure 5.

Toutefois, comme il existe d'autres moyens pour exciter une surface activable, la pointe 21 n'est pas nécessairement conductrice. Elle peut être par exemple en plastique, et apte à maintenir une charge sur la surface 2 de manière à exciter ladite surface activable.

Comme illustré également en figure 5, lorsque la pointe 21 touche la surface 2 activable d'un système 3 apte à contenir des données, tel qu'une tablette, un smartphone ou un ordinateur, elle permet la réception et la transmission d'informations entre ledit système et le dispositif de retour haptique 1.

Par exemple, la pointe 21 permet de transmettre au système 3 la position x, y du point de contact 4, le système 3 fournissant en retour des informations sur ledit point de contact (informations sur le relief, telles une épaisseur, une hauteur et/ou toute autre information permettant de caractériser le relief, et informations sur la raideur).

Les informations sur le relief sont transmises au premier actionneur 30 de manière à ce qu'il réagisse en appliquant aux moyens de contact 20, via la pointe 21 en contact avec la surface 2 dans l'exemple illustré, un mouvement de translation par rapport au corps 10. L'amplitude et le sens de ce mouvement de translation sont fonction des informations sur le relief transmises par le système 3, informations qui sont transformées en une valeur z et/ou en une autre valeur. La pointe 21 restant en contact avec la surface 2, c'est le corps 10 qui va se déplacer selon ladite valeur z :
- soit en s'enfonçant dans la main (sens A), par exemple pour simuler une bosse, une convexité ...
- soit en s'extrayant de la main (sens B), par exemple pour simuler un creux, une concavité ...

Comme représenté en figures 2 et 5, les moyens de contact 20 sont configurés pour faire des mouvements d'aller et de retour en translation par rapport au corps 10, une première partie 20a desdits moyens de contact étant aptes à coulisser essentiellement à l'intérieur dudit corps et une seconde partie 20b étant aptes à coulisser à l'intérieur et à l'extérieur dudit corps. La seconde partie 20b comprend la pointe 21. Le corps 10 présente une ouverture 12 apte à permettre le passage les moyens de contact 20 entre l'intérieur et l'extérieur dudit corps 10.

Comme illustré, le premier actionneur 30 comprend un premier moteur 31 linéaire. Ledit premier moteur 31 est relié aux moyens de contact 20 et il permet d'appliquer un mouvement de translation auxdits moyens de contact 20 par rapport au corps 10. Un moteur linéaire a l'avantage de s'adapter aisément à la forme allongée du dispositif, ce qui en facilite notamment l'assemblage.

Alternativement, le premier moteur 31 peut fonctionner en rotation, et être associé à des moyens mécaniques (vis à billes, système de transmission) permettant de transformer le mouvement de rotation en un mouvement de translation. L'avantage d'un moteur en rotation est qu'il est en général moins encombrant.

Les informations sur la raideur sont transmises au second actionneur 50 pour qu'il agisse sur les moyens de freinage 40 de sorte que ledit second actionneur fasse varier la résistance mécanique appliquée par lesdits moyens de freinage 40 aux mouvements entre les moyens de contact 20 et le corps 10 du dispositif. Elle est par conséquent ressentie par la main 5 de l'utilisateur en contact avec le corps 10. Cette résistance mécanique ressentie par l'utilisateur est fonction des informations sur la raideur transmises par le système 3, informations qui sont transformées en une valeur ϕ et/ou en une autre valeur.

Comme illustré, les moyens de freinage 40 comprennent un ressort 41 qui permet d'appliquer une résistance mécanique variable en fonction de la raideur dudit ressort. Le second actionneur 50 comprend un second moteur 51 relié audit ressort et qui agit en augmentant ou en raccourcissant la longueur dudit ressort. Ce faisant, il diminue ou il augmente la raideur dudit ressort. Cette raideur est appliquée au mouvement entre la pointe 21 et le corps 10 du dispositif, lorsque la pointe 21 est en contact avec la surface 2.

Comme illustré, le second moteur 51 fonctionne en rotation et il peut donc tourner selon l'angle ϕ. Un moteur en rotation permet de réduire l'encombrement. En outre, il permet une meilleure précision.

Alternativement, le second moteur peut fonctionner en translation, ou selon toute autre cinématique permettant de modifier la longueur, et pas conséquent la raideur du ressort.

Comme illustré, le premier actionneur 30 et les moyens de freinage 40 couplés au second actionneur 50 sont disposés en série. Le premier moteur 31 est relié au ressort 41 par des premiers moyens de liaison 34 qui comprennent une tige en T, la partie longitudinale du T étant couplée au premier moteur 31 et la partie transversale du T étant couplée au ressort 41. La partie transversale du T est retenue par une couronne rigide 15 fixée transversalement à l'intérieur du corps 10, de sorte que la longueur du ressort ne peut pas dépasser une certaine longueur. En d'autres termes, la couronne rigide 15 permet de définir une référence de la base du ressort par rapport au corps 10.

Cette configuration en série permet de limiter l'encombrement dans la direction transversale du dispositif de retour haptique. Elle permet au corps 10 d'avoir une forme allongée, comme cela est requis lorsque le corps 10 présente une forme de stylet, comme illustré dans les figures suivantes.

Alternativement, le premier actionneur 30 et les moyens de freinage 40 couplés au second actionneur 50 peuvent être disposés en parallèle.

Les figures 3A et 3B illustrent le même mode de réalisation, avec plus de détails, et selon une vue en coupe et une vue en 3D. La figure 4 illustre le même mode vu de l'extérieur.

Le corps 10 comprend une coque 13, visible en figure 4. La coque 13 peut être en un matériau apte à supporter le poids des composants qu'elle contient, par exemple en plastique résistant. Ladite coque contient les premier et second actionneurs 30, 50 ainsi que les moyens de freinage 40.

Le corps 10 présente une ouverture 12 de sorte que les moyens de contact 20 puissent traverser ledit corps.

Le corps 10 peut comprendre également une pièce d'extrémité 14. L'ouverture 12 peut être réalisée dans ladite pièce d'extrémité. Le corps peut donc ne pas être monolithique : par exemple, la pièce d'extrémité 14 peut être en un matériau conducteur et le reste du corps 10 peut être en un matériau isolant. La pièce d'extrémité 14 peut être raccordée à la coque 13, par exemple clipsée ou encastrée, ou par un système de filetage.

Les moyens de contact 20 sont en partie contenus dans le corps 10 et comprennent une pointe 21 reliée à une tige 23. La pointe 21 présente un diamètre inférieur à la tige 23. L'ouverture 12 du corps 10 présente un diamètre supérieur au diamètre de la pointe 21 mais inférieur au diamètre de la tige 23, de sorte que seule ladite pointe puisse rentrer et sortir dudit corps.

Le dispositif illustré présente la forme d'un stylet.

Le premier actionneur 30 comprend un premier moteur 31 linéaire, comme décrit en lien avec la figure 2. Le premier actionneur 30 est relié aux moyens de contact 20 par l'intermédiaire d'une liaison 32 de type pivot, ladite liaison 32 étant maintenue par une goupille 33.

Les moyens de freinage 40 comprennent un ressort 41, comme décrit en lien avec la figure 2.

Le second actionneur 50 comprend un second moteur 51 rotatif, comme décrit en lien avec la figure 2.

Le mouvement du dispositif 1 est réalisé par l'intermédiaire des moyens de contact 20 et plus précisément par la pointe 21 desdits moyens de contact (qui est donc également la pointe du stylet). Ladite pointe est actionnée par le premier moteur 31, mais également par le ressort 41 dont la raideur est réglée par le moteur 51. Le moteur 31 et le ressort 41 couplé au moteur 51 sont montés en série l'un après l'autre. Le premier moteur 31 est relié au ressort 41 par les moyens de liaison 34, précédemment décrits.

Le premier moteur 31, qui peut être désigné comme « actionneur linéaire », sert à transmettre les informations de relief, et le second moteur 51 rotatif, qui peut être désigné comme « servomoteur », est couplé au ressort 41, qui sert à transmettre les informations de raideur. Plus la surface réelle correspondant au point de contact 4 sera dure, plus le ressort sera comprimé par le servomoteur, la pointe 21 aura alors une résistance mécanique plus importante contre la surface 2 en contact avec le stylet. Et inversement, moins la surface réelle correspondant au point de contact 4 sera dure, moins le ressort sera comprimé par le servomoteur, la pointe 21 aura alors une résistance mécanique moins importante contre la surface 2 en contact avec le stylet

Dans l'exemple illustré, la transmission du mouvement de sortie en rotation du servomoteur 51 au ressort 41 est réalisée par des seconds moyens de liaison 52, par exemple une liaison de type pignon-crémaillère. La liaison 52 de type pignon-crémaillère permet de transformer le mouvement de rotation du servomoteur 51 en une translation appliquée au ressort 41. Le pignon 52b est relié au servomoteur 51 et la crémaillère 52a est reliée au ressort 41 de sorte que celui-ci se comprime ou se détend sous l'action dudit servomoteur.

La raideur du ressort est alors prise en compte par l'actionneur linéaire 31 qui, lui, déplace la pointe 21 selon un mouvement de translation par rapport au corps 10. Le mouvement de translation appliqué à la pointe 21 est assorti d'une résistance mécanique plus ou moins importante, selon la raideur du ressort appliquée.

La pointe 21 étant en contact avec la surface 2, c'est le corps du stylet 1 qui reçoit ces mouvements et les transmet à la main de l'utilisateur en contact avec l'extrémité 12 du stylet

Ainsi, l'actionneur linéaire 31 permet de transmettre physiquement à un utilisateur l'information de relief correspondant au point de contact 4, et le couple servomoteur 51-ressort 41 permet de transmettre physiquement à un utilisateur l'information de raideur associée audit relief.

Avec un dispositif selon l'invention, il est possible de ressentir des reliefs de quelques millimètres pour une surface balayée de quelques centimètres carrés, ce qui est déjà amplement suffisant pour certains utilisateurs qui doivent effectuer des gestes minutieux et précis.

La « surface balayée » représente typiquement la taille de l'image d'un objet vu sur un écran. L'image vue sur l'écran et qui doit être « ressentie » avec le dispositif haptique selon l'invention peut avoir une échelle adaptée à l'application. Pour la microchirurgie, l'image représentée un objet « grossi » pour cibler mieux l'objectif. Par exemple, sur un écran d'une tablette tactile de 20cm x 30cm, il peut être courant d'avoir l'image d'un objet dont la taille réelle est par exemple de 2cm x 3cm.

On peut utiliser des actionneurs et de moyens de freinage de petites tailles, c'est-à-dire de quelques centimètres et les configurer entre eux pour que le dispositif de retour haptique ait la dimension d'un stylet (de l'ordre de 10 cm sur 3 cm). A titre d'exemple :
- l'actionneur 30 peut être actionneur linéaire de type Firgelli (modèle : PQ12-100-6) dont les dimensions extérieures sont 21.5 mm x 15 mm x 36.5 mm ;
- les moyens de freinage 40 peuvent être un ressort de compression dont les dimensions sont φ = 9.4 mm et L=16 mm ;
- l'actionneur 50 peut être moteur de type servomoteur de marque UltraNano de Hitec (modèle HS-35HD) dont les dimensions extérieures sont 18.6 mm x 15.5 mm x 7.6 mm ;
- l'alimentation 60 est une source de voltage de 6 Vdc : elle peut être une entrée extérieure ou une batterie 62 disposée à l'intérieur du corps 10 (illustrée en figure 5) ;
- les cartes 70 sont des cartes standards destinées au contrôle des deux moteurs ainsi qu'à la communication avec le système 3.

Les amplitudes de translation peuvent également être dimensionnées, par exemple en déterminant une longueur de pointe, en déterminant une longueur de stylet et en modifiant si nécessaire le premier actionneur et notamment sa course.

Les valeurs de résistance mécanique applicables peuvent aussi être aisément modifiables, en modifiant par exemple le type de ressort ou en remplaçant un ressort par un autre moyen.

Ainsi, le dispositif selon l'invention est très modulable, aisément ajustable en fonction des objets et de l'environnement à reproduire.

Le dispositif comprend en outre des moyens d'alimentation 60, aptes à alimenter électriquement l'actionneur linéaire 31 et le servomoteur 51. Comme illustré, les moyens d'alimentation peuvent comprendre un port de connexion 61 apte à coopérer avec une prise reliée à une alimentation extérieure au stylet.

La figure 5 illustre et précise le fonctionnement d'un dispositif selon le même mode de réalisation.

Comme illustré, le dispositif de retour haptique selon l'invention peut comprendre des moyens de traitement 70.

Lesdits moyens de traitement 70 peuvent être sous la forme d'une ou de plusieurs cartes électroniques dont certaines peuvent être disposées dans le corps 10 du dispositif, par exemple :
- au moins une carte de communication 71 apte à établir un protocole de communication entre le dispositif 1 et le système 3, notamment pour échanger les informations de coordonnées x, y du point de contact 4, et les informations (sur le relief ou sur la raideur) dudit système en ledit point de contact : une première carte de communication 71a peut être dans le dispositif 1 et une seconde carte de communication 71b peut être au niveau du système 3 ;
- une carte de calcul 72 pour transformer les informations en valeurs, par exemple en valeur z pour le relief, et en valeur ϕ pour la raideur : elle peut être dans le dispositif 1 ou au niveau du système 3 (comme représenté) ;
- une carte de contrôle/commande 73 apte à commander le premier actionneur 30 et le second actionneur 50, par exemple pour transmettre au premier actionneur 30 une commande de translation selon la valeur z et au second actionneur 50 une commande de rotation selon la valeur ϕ afin de modifier la résistance appliquée par les moyens de freinage 40, en fonction des informations transmises par le système 3 pour le point de contact 4 : elle peut être dans le dispositif 1 (comme représenté) ;
- une carte de puissance 74 pour que ces actionnements puissent être effectués : elle peut être dans le dispositif 1 (comme représenté).

Sur l'exemple représenté, la carte de puissance 74 est alimentée par une batterie 62.

Alternativement, il peut s'agir de moyens de communication et/ou de moyens de calcul et/ou de moyens de contrôle/commande et/ou de moyens de puissance qui ne sont pas nécessairement des cartes, mais qui assurent les mêmes fonctions.

Le fonctionnement du dispositif de retour haptique selon l'exemple représenté est donc le suivant :
- la pointe 21 des moyens de contact 20 vient en contact en un point 4 avec la surface activable 2 d'un système 3, de manière à exciter ladite surface et à ce que le système 3 localise les coordonnées x,y du point de contact 4 ;
- le système 3 renvoie des informations correspondant aux coordonnées x,y du point de contact 4 : hauteur, épaisseur et/ou toute autre information permettant de caractériser le relief et toute information permettant de caractériser la raideur ;
- les informations renvoyées sont transformées en valeurs (z pour le relief et ϕ pour la raideur) ;
- la valeur z est transmise à l'actionneur linéaire (premier moteur 31) pour qu'il applique un mouvement de translation z aux moyens de contact 20 par rapport au corps 10 ;
- la valeur ϕ est transmise au servo-moteur (second actionneur 51) pour qu'il effectue une rotation ϕ afin de modifier la résistance mécanique du ressort 41 ;
- ensuite, l'utilisateur peut déplacer le dispositif de retour haptique sur la surface 2 et le fonctionnement précédemment décrit est réitéré.

Le système 3 apte à contenir des informations peut être une tablette et la surface 2 un écran tactile de la tablette.

La présente invention n'est pas limitée au mode de réalisation précédemment décrit mais s'étend à tout mode de réalisation entrant dans la portée des revendications.

L'invention peut trouver de nombreuses applications, notamment dans les domaines de :
- la chirurgie robotisée, domaine dans lequel il est nécessaire d'avoir la précision la plus élevée possible, particulièrement dans le domaine de la microchirurgie assistée ;
- l'imagerie médicale, qui permet notamment d'enregistrer des informations dans un système informatique, lesquelles informations pouvant être parcourues à distance par un utilisateur ;
- la conception assistée par ordinateur, notamment pour le design, la fabrication de mobiliers, pour ressentir les formes, le confort...
- la téléopération, notamment dans les domaines du nucléaire pour lesquelles les opérations se font à distance ;
- l'activité de soutien à la rééducation ;
- l'aide aux malvoyants ;
- les jeux, notamment les jeux vidéo...,
et plus généralement dans tous les cas où l'on souhaite créer une interface matérielle active indirecte entre la main d'un utilisateur et un environnement réel, de réalité augmentée ou virtuelle ou un objet.

## Revendications

1. Dispositif de retour haptique comprenant :
- un corps (10) apte à former une interface avec un utilisateur ;
- des moyens de contact (20) aptes à établir un contact avec une surface (2) d'un système (3) apte à contenir des informations ;
- un premier actionneur (30) relié aux moyens de contact (20) et apte à appliquer un mouvement de translation auxdits moyens de contact par rapport audit corps ; et
**caractérisé en ce qu'**il comprend, en outre :
- des moyens de freinage (40) aptes à appliquer une résistance mécanique au mouvement relatif entre les moyens de contact (20) et le corps (10) ;
- un second actionneur (50) apte à faire varier la résistance mécanique appliquée par les moyens de freinage (40).

2. Dispositif selon la revendication 1, ayant sensiblement la forme d'un stylet.

3. Dispositif selon la revendication 1 ou 2, le corps (10) présentant au moins une partie conductrice (14).

4. Dispositif selon l'une des revendications 1 à 3, le corps (10) étant apte à contenir tout ou partie du premier actionneur (30) et/ou des moyens de freinage (40) et/ou du second actionneur (50).

5. Dispositif selon l'une des revendications 1 à 4, le corps (10) comprenant une coque (13) apte à contenir le premier actionneur (30), le second actionneur (50), et les moyens de freinage (40).

6. Dispositif selon l'une des revendications précédentes, les moyens de contact (20) comprenant une pointe (21) dont une première extrémité (22) est apte à établir un contact avec la surface (2) du système (3).

7. Dispositif selon la revendication 6, la première extrémité (22) de la pointe (21) étant apte à exciter la surface (2) du système (3).

8. Dispositif selon l'une des revendications précédentes, le premier actionneur (30) comprenant un moteur linéaire (31).

9. Dispositif selon l'une des revendications précédentes, comprenant des premiers moyens de liaison (34) aptes à relier le premier actionneur (30) aux moyens de contact (20).

10. Dispositif selon l'une des revendications précédentes, comprenant des seconds moyens de liaison (52) aptes à relier le second actionneur (50) aux moyens de freinage (40)

11. Dispositif selon l'une des revendications précédentes, les moyens de freinage (40) comprenant un ressort (41).

12. Dispositif selon la revendication 11, le second actionneur (50) étant apte à faire varier la raideur du ressort (41) en faisant varier la longueur dudit ressort.

13. Dispositif selon l'une des revendications précédentes, le second actionneur (50) comprenant un moteur (51) rotatif.

14. Dispositif selon la revendication 13, les moyens de liaison (52) comprenant une liaison de type pignon - crémaillère.

15. Dispositif selon l'une des revendications précédentes comprenant en outre des moyens d'alimentation (60), aptes à connecter électriquement les premier et second actionneurs dudit dispositif.

16. Dispositif selon la revendication 15, les moyens d'alimentation (60) comprenant une batterie (62).

17. Dispositif selon la revendication 15 ou 16, les moyens d'alimentation (60) comprenant une interface de branchement (61) avec une alimentation extérieure.

18. Dispositif selon l'une des revendications 15 à 17, les moyens d'alimentation (60) étant aptes à activer électriquement les moyens de contact (20).

19. Dispositif selon l'une des revendications précédentes comprenant en outre des moyens de traitement (70) aptes à activer le premier actionneur (30) et le second actionneur (50) en fonction de la position des moyens de contacts (20) sur la surface (2).

20. Dispositif selon l'une des revendications précédentes, dans lequel le premier actionneur (30) et les moyens de freinage (40) couplés au second actionneur (50) sont disposés en série.

## Patentansprüche

1. Vorrichtung mit haptischer Rückmeldung, aufweisend:
- einen Körper (10), der geeignet ist, eine Schnittstelle für einen Benutzer zu bilden;
- ein Kontaktmittel (20), das ausgebildet ist einen Kontakt mit einer Oberfläche (2) eines Systems (3) herzustellen, welches zur Aufnahme von Informationen ausgebildet ist;
- einen ersten Aktuator (30), der mit dem Kontaktmittel (20) verbunden ist und geeignet ist, eine Translationsbewegung des Kontaktmittels relativ zum Körper zu bewirken; und
**dadurch gekennzeichnet, dass** die Vorrichtung mit haptischer Rückmeldung außerdem aufweist:
- ein Bremsmittel (40), das ausgebildet ist der Relativbewegung zwischen dem Kontaktmittel (20) und dem Körper (10) einen mechanischen Widerstand entgegenzusetzen;
- einen zweiten Aktuator (50), der ausgebildet ist den von dem Bremsmittel (40) ausgeübten mechanischen Widerstand zu ändern.

2. Vorrichtung nach Anspruch 1, wobei sie im Wesentlichen die Form eines Stifts aufweist.

3. Vorrichtung nach Anspruch 1 oder 2, wobei der Körper (10) mindestens einen leitenden Abschnitt (14) aufweist.

4. Vorrichtung nach einem der Ansprüche 1 bis 3, wobei der Körper (10) ausgebildet ist den ersten Aktuator (30) und/oder das Bremsmittel (40) und/oder den zweiten Aktuator (50) ganz oder teilweise aufzunehmen.

5. Vorrichtung nach einem der Ansprüche 1 bis 4, wobei der Körper (10) ein Gehäuse (13) aufweist, das ausgebildet ist den ersten Aktuator (30), den zweiten Aktuator (50) und das Bremsmittel (40) aufzunehmen.

6. Vorrichtung nach einem der vorherigen Ansprüche, wobei das Kontaktmittel (20) eine Spitze (21) aufweist, deren erstes Ende (22) dazu ausgebildet ist einen Kontakt mit der Oberfläche (2) des Systems (3) herzustellen.

7. Vorrichtung nach Anspruch 6, wobei das erste Ende (22) der Spitze (21) dazu ausgebildet ist die Oberfläche (2) des Systems (3) anzuregen.

8. Vorrichtung nach einem der vorherigen Ansprüche, wobei der erste Aktuator (30) einen Linearmotor (31) aufweist.

9. Vorrichtung nach einem der vorherigen Ansprüche, aufweisend ein Verbindungsmittel (34), das ausgebildet ist den ersten Aktuator (30) mit dem Kontaktmittel (20) zu verbinden.

10. Vorrichtung nach einem der vorherigen Ansprüche, aufweisend ein zweites Verbindungsmittel (52), das ausgebildet ist den zweiten Aktuator (50) mit dem Bremsmittel (40) zu verbinden

11. Vorrichtung nach einem der vorherigen Ansprüche, wobei das Bremsmittel (40) eine Feder (41) aufweist.

12. Vorrichtung nach Anspruch 11, wobei der zweite Aktuator (50) dazu ausgebildet ist die Federkraft der Feder (41) zu verändern, indem er den Federweg der Feder verstellt.

13. Vorrichtung nach einem der vorherigen Ansprüche, wobei der zweite Aktuator (50) einen rotierenden Motor (51) aufweist.

14. Vorrichtung nach Anspruch 13, wobei das Verbindungsmittel (52) eine Zahnstangen-Ritzel-Verbindung aufweist.

15. Vorrichtung nach einem der vorherigen Ansprüche, aufweisend eine Energieversorgungseinrichtung (60), die ausgebildet ist den ersten und den zweiten Aktuator der Vorrichtung elektrisch zu verbinden.

16. Vorrichtung nach Anspruch 15, wobei die Energieversorgungseinrichtung (60) eine Batterie (62) aufweist.

17. Vorrichtung nach Anspruch 15 oder 16, wobei die Energieversorgungseinrichtung (60) eine Anschlussschnittstelle (61) zu einer externen Energieversorgung aufweist.

18. Vorrichtung nach einem der Ansprüche 15 bis 17, wobei die Energieversorgungseinrichtung (60) ausgebildet ist das Kontaktmittel (20) elektrisch zu aktivieren.

19. Vorrichtung nach einem der vorherigen Ansprüche, aufweisend eine Recheneinheit (70), die ausgebildet ist den ersten Aktuator (30) und den zweiten Aktuator (50) in Abhängigkeit von der Position des Kontaktmittels (20) auf der Oberfläche (2) zu aktivieren.

20. Vorrichtung nach einem der vorherigen Ansprüche, wobei der erste Aktuator (30) und das mit dem zweiten Aktuator (50) gekoppelte Bremsmittel (40) in Reihe angeordnet sind.

## Claims

1. A haptic feedback device comprising:
- a body (10) able to form an interface with a user;
- contacting means (20) able to establish a contact with a surface (2) of a system (3) able to contain information;
- a first actuator (30) linked to the contacting means (20) and able to apply a translational movement to said contacting means with respect to said body; **characterized in that** it further comprises:
- braking means (40) able to apply a mechanical resistance to the relative movement between the contacting means (20) and the body (10);
- a second actuator (50) able to make the mechanical resistance applied by the braking means (40) vary.

2. The device as claimed in claim 1, having substantially the form of a stylus.

3. The device as claimed in claim 1 or 2, the body (10) having at least one conductive portion (14).

4. The device as claimed in one of claims 1 to 3, the body (10) being able to contain all or some of the first actuator (30) and/or of the braking means (40) and/or of the second actuator (50).

5. The device as claimed in one of claims 1 to 4, the body (10) comprising a casing (13) able to contain the first actuator (30), the second actuator (50), and the braking means (40).

6. The device as claimed in one of the preceding claims, the contacting means (20) comprising a tip (21) with a first end (22) able to establish a contact with the surface (2) of the system (3).

7. The device as claimed in claim 6, the first end (22) of the tip (21) being able to excite the surface (2) of the system (3).

8. The device as claimed in one of the preceding claims, the first actuator (30) comprising a linear motor (31).

9. The device as claimed in one of the preceding claims, comprising first linking means (34) able to link the first actuator (30) to the contacting means (20).

10. The device as claimed in one of the preceding claims, comprising second linking means (52) able to link the second actuator (50) to the braking means (40).

11. The device as claimed in one of the preceding claims, the braking means (40) comprising a spring (41).

12. The device as claimed in claim 11, the second actuator (50) being able to make the stiffness of the spring (41) vary by making the length of said spring vary.

13. The device as claimed in one of the preceding claims, the second actuator (50) comprising a rotary motor (51).

14. The device as claimed in claim 13, the linking means (52) comprising a link of rack-and-pinion type.

15. The device as claimed in one of the preceding claims, furthermore comprising powering means (60) able to electrically connect the first and second actuators of said device.

16. The device as claimed in claim 15, the powering means (60) comprising a battery (62).

17. The device as claimed in claim 15 or 16, the powering means (60) comprising an interface (61) for plugging into an exterior power supply.

18. The device as claimed in one of claims 15 to 17, the powering means (60) being able to electrically activate the contacting means (20).

19. The device as claimed in one of the preceding claims, furthermore comprising processing means (70) able to activate the first actuator (30) and the second actuator (50) depending on the position of the contacting means (20) on the surface (2).

20. The device as claimed in one of the preceding claims, wherein the first actuator (30) and the braking means (40) coupled to the second actuator (50) are placed in series.
